# EUROPÄISCHE PATENTSCHRIFT

(11) **EP 3 817 691 B1**
(45) Veröffentlichungstag und Bekanntmachung des Hinweises auf die Patenterteilung: **25.09.2024**
(21) Anmeldenummer: 19791198.5
(22) Anmeldetag: 17.10.2019
(51) Int. Cl.: A61F 2/52, A41C 3/14

(54) **VERFAHREN ZUR ANWENDERSEITIGEN VOLUMENANPASSUNG EINER BRUSTPROTHESE**
METHOD FOR USER-SIDE VOLUME ADAPTATION OF A BREAST PROSTHESIS
PROCÉDÉ PERMETTANT À UNE UTILISATRICE D'ADAPTER LE VOLUME D'UNE PROTHÈSE MAMMAIRE

(30) Priorität: 19.10.2018 DE 102018126035
(43) Veröffentlichungstag der Anmeldung: 12.05.2021
(73) Patentinhaber: Amoena Medizin-Orthopädie-Technik GmbH, 83064 Raubling (DE)
(72) Erfinder: STELTER, Nils, 83112 Frasdorf (DE); WILD, Helmut, 83071 Stephanskirchen (DE)
(74) Vertreter: Laufhütte, Dieter
(86) Internationale Anmeldenummer: PCT/EP2019/078259
(87) Internationale Veröffentlichungsnummer: WO 2020/079172

(56) Entgegenhaltungen:
- DE-B- 1 008 224
- US-A- 2 516 129
- US-A- 2 698 436
- US-B1- 6 936 068

## Beschreibung

Die Erfindung betrifft ein Verfahren zur anwenderseitigen Volumenanpassung einer Brustprothese.

Brustprothesen werden nach operativen Brustentfernungen getragen. Anforderungen an die Brustprothesen umfassen insbesondere eine der natürlichen Brust möglichst nahekommenden Form und Haptik sowie ein hoher Tragekomfort.

Um ohne kostspielige Individualanfertigungen erreichen zu können, dass das Volumen einer derartigen Prothese an die individuellen Bedürfnisse der Trägerin angepasst werden kann, die sich aus der Größe der noch gesunden Brust, sofern vorhanden, oder aus dem persönlichen Wohlbefinden ableiten, wurde bereits im Stand der Technik vorgeschlagen, Brustprothesen bereitzustellen, die in ihrem Volumen nachträglich angepasst werden können. Als Beispiel hierfür ist etwa die EP 0 824 001 A2 zu nennen.

Aus der US 2516 129 A, die als nächstliegender Stand der Technik angesehen wird, ist grundsätzlich bereits ein Verfahren zur Volumenanpassung einer Brustprothese bekannt. Die US 2698 436 A beschreibt einen Büstenhalter mit Einlagen, wobei die Einlage fest mit dem Büstenhalter verbunden ist.

Aufgabe der Erfindung ist es, ein Verfahren zur anwenderseitigen Volumenanpassung einer dafür geeigneten Brustprothese bereitzustellen.

Vor diesem Hintergrund betrifft die Erfindung ein Verfahren zur Volumenanpassung einer Brustprothese, wobei die Brustprothese einen ersten Schalenkörper, einen damit umlaufend verbundenen zweiten Schalenkörper und einen zwischen den Schalenkörpern angeordneten Fluidraum aufweist, wobei der Fluidraum durch einen mit einem Ventil versehenen Durchgang von der Außenseite her zugänglich ist, wobei das Verfahren die folgenden Schritte aufweist: (a) Einlegen der Brustprothese in einen von einer Patientin getragenen Büstenhalter; (b) Befüllen des Fluidraums mit einem Fluid oder Entnehmen eines Fluids aus dem Fluidraum, während die Brustprothese in den von der Patientin getragenen Büstenhalter eingelegt ist.

Bei den Schalenkörpern der erfindungsgemäß verwendeten Brustprothese handelt es sich vorzugsweise um mit einem verformbaren Material gefüllte Folienbeutel. Bei dem verformbaren Material handelt es sich vorzugsweise um einen vernetzten Silikonkautschuk, vorzugsweise Zwei-Komponenten-Silikonkautschuk. Bei den Folien des Folienbeutels handelt es sich vorzugsweise um Kunststofffolien. Vorzugsweise sind wenigstens vier Folien entlang einem gemeinsamen Umfang verbunden, insbesondere verschweißt. Dieser Aufbau erlaubt es den Schalenkörpern die Anforderungen an Haptik und Tragekomfort zu erfüllen und eine Expansion des Fluidraumvolumens bei dessen Befüllen mit einem Fluid oder eine Schrumpfung des Fluidraumvolumens bei der Entnahme von Fluid nicht zu behindern.

Der Silikonkautschuk der Schalenkörper kann zusätzliche Bestandteile wie beispielsweise ein Phasenwechselmaterial oder poröses Granulat bzw. Hohlkugeln umfassen. Phasenwechselmaterialien dienen zur Verbesserung der Wärmeregulierung an der Haut der Trägerin und werden daher bevorzugt dem Silikonkautschuk des ersten Schalenkörpers beigemengt, der in der Anwendung auf Seiten der Trägerin liegen soll. Geeignete Phasenwechselmaterialien umfassen solche, deren Phasenübergangstemperatur nahe der Körpertemperatur liegt. Beispiele umfassen Paraffine mit einer geeigneten Zahl an Kohlenstoffatomen, typischerweise etwa zwanzig, um einen Schmelzpunkt im gewünschten Bereich einzustellen. Granulate bzw. Hohlkugeln können dazu dienen, das Gewicht der Prothese zu verringern, ohne die haptischen Eigenschaften zu beeinträchtigen.

Bei dem Fluid zur Volumenanpassung kann es sich um ein Gas, insbesondere um Luft handeln. In dieser Ausführungsform kann eine einfache Luftpumpe zur Volumenanpassung verwendet werden.

Alternativ kann es sich bei dem Fluid zur Volumenanpassung um eine Flüssigkeit, insbesondere um eine nachträglich vernetzbare viskose Flüssigkeit handeln. Ein besonders geeignetes Beispiel umfasst ein vernetzbares Silikon-Fluid. In diesem Fall kann das Verfahren ferner einen Schritt des nachträglichen Vernetzens der in den Fluidraum eingefüllten Flüssigkeit umfassen. Durch die Verwendung und nachträgliche Vernetzung kann erreicht werden, dass die Füllung des Hohlraums mit den Schalenkörpern vergleichbare physikalische Eigenschaften aufweist, was sich insgesamt positiv auf die haptischen Eigenschaften der Prothese auswirken kann. Der Schritt des nachträglichen Vernetzens erfolgt in wenigen Minuten bei Raumtemperatur im Büstenhalter. Zu beachten ist, dass ein vernetztes Fluid nicht mehr nachträglich entnommen werden kann, um das Volumen zu verringern.

Auch Kombinationen von Gas und Flüssigkeit können als Fluid zur Volumenanpassung in einer Ausgestaltung der Erfindung zur Anwendung kommen.

Im Rahmen des Schrittes (a) kann vorgesehen sein, dass die volumenanpassbare Brustprothese zwischen die Haut der Patientin und den Cup des Büstenhalters gelegt wird. Im Falle eines Büstenhalters mit integrierten Taschen kann alternativ vorgesehen sein, dass die Brustprothese im Rahmen des Schrittes (a) in die betreffende Tasche des Büstenhalters gelegt wird.

In einer Ausführungsform ist vorgesehen, dass sich innerhalb des Fluidraums ein von dem in Schritt (b) einzufüllenden Fluid zur Volumenanpassung verschiedenes, zusätzliches Medium befindet, welches die Adhäsionsneigung der gegenüberliegenden Innenoberflächen der Schalenkörper verringert. Die erfindungsgemäße nachträgliche Volumenanpassung kann dadurch behindert werden, dass die Innenoberflächen der Schalenkörper aneinander haften bleiben.

Auch eine permanente Deformierung der Brustprothese durch ein derartiges Anhaften wäre zu befürchten. Durch das zusätzliche Medium wird die Tendenz für eine solche unerwünschte Adhäsion verringert.

Bei dem Medium zur Verringerung der Adhäsionsneigung der gegenüberliegenden Innenoberflächen der Schalenkörper kann es sich um eine Flüssigkeit, insbesondere um ein Öl und weiter bevorzugt um ein Silikon-Öl handeln. In dem Fall, dass es sich sowohl bei dem Fluid zur Volumenanpassung als auch bei dem Medium zur Verringerung der Adhäsionsneigung der gegenüberliegenden Innenoberflächen der Schalenkörper jeweils um eine Flüssigkeit handelt, unterscheiden sich diese Flüssigkeiten voneinander.

Ferner kann es sich bei dem Medium zur Verringerung der Adhäsionsneigung der gegenüberliegenden Innenoberflächen der Schalenkörper um einen pulverförmigen Feststoff handeln. Die durchschnittliche Partikelgröße der Pulverkörner liegt vorzugsweise im Bereich von zwischen 1 nm bis 1 mm.

Bei dem Medium zur Verringerung der Adhäsionsneigung der gegenüberliegenden Innenoberflächen der Schalenkörper kann es sich gegebenenfalls auch um ein Gas handeln. In dem Fall, dass es sich sowohl bei dem Fluid zur Volumenanpassung als auch bei dem Medium zur Verringerung der Adhäsionsneigung der gegenüberliegenden Innenoberflächen der Schalenkörper jeweils um ein Gas handelt, unterscheiden sich diese Gase voneinander.

Auch Kombinationen aus den genannten Medien zur Verringerung der Adhäsionsneigung der gegenüberliegenden Innenoberflächen der Schalenkörper kommen in weiteren Ausführungsformen zur Anwendung. Beispielsweise kann ein Gemisch aus Flüssigkeiten und dispergierten pulverförmigen Feststoffen zur Anwendung kommen, wobei die Flüssigkeiten und dispergierten pulverförmigen Feststoffe wie oben definiert ausgebildet sein können.

Bei dem Durchgang kann es sich um einen Ventilschlauch handeln, der vorzugsweise in radialer Richtung durch den Verbindungsbereich zwischen den beiden Schalenkörpern verläuft. Der Schlauch ist vorzugsweise zwischen den Folien der Folienbeutel eingeklebt oder eingeschweißt und durchläuft die Schweiß- oder Klebenaht zwischen den Folienbeuteln so in radialer Richtung. Der Ventilschlauch besteht vorzugsweise aus einem flexiblen Material und insbesondere aus einem flexiblen Kunststoffmaterial. Es kann vorgesehen sein, dass der Schlauch über den Verbindungsbereich hinaus in den Fluidraum ragt. Der Ventilschlauch weist generell ein Rückschlagventil wie beispielsweise ein Flatterventil auf.

Es kann vorgesehen sein, dass zum Befüllen oder Entnehmen des Fluidraums eine Nadel in den Ventilschlauch gesteckt wird und das Fluid zur Volumenanpassung durch diese Nadel in den Fluidraum gedrückt wird. Die Nadel kann beispielsweise aus Kunststoff oder Metall gefertigt sein. Vorzugswiese ist die Nadel stumpf, um das Ventil, beispielsweise die Membran des Flatterventils nicht zu beschädigen.

In einer Variante ist vorgesehen, dass der Ventilschlauch an seiner Innenseite eine Stufe umfasst, an der sich dessen Innendurchmesser in Richtung zum Fluidraum hin verringert, und dass vorzugsweise die Nadel eine korrespondierende Stufe an der Außenseite aufweist, an der sich deren Innendurchmesser in Richtung zum Fluidraum hin verringert. Jede der Stufen kann individuell eine gerade, d.h. normal auf die Schlauch- bzw. Nadelachse stehend Stufenfläche oder eine geneigte, d.h. schräg auf die die Schlauch- bzw. Nadelachse stehende Stufenfläche. Die korrespondierenden Stufen können als Anschlag dienen, um ein zu tiefes Eindringen der Nadel zu verhindern.

Im Rahmen des Verfahrens kann die Brustprothese vor Schritt (a) oder zwischen den Schritten (a) und (b) mit einer Befüll- und/oder Entnahmevorrichtung verbunden werden. Die Befüll- und/oder Entnahmevorrichtung kann beispielsweise eine Pumpe, einen Schlauch und eine Nadel aufweisen. In einer Ausführungsform kann die Brustprothese also bereits mit einer Befüll- und/oder Entnahmevorrichtung verbunden sein, bevor diese in den Büstenhalter eingelegt wird. Alternativ kann vorgesehen sein, dass die Brustprothese erst dann mit einer Befüll- und/oder Entnahmevorrichtung verbunden wird, nachdem diese in den Büstenhalter eingelegt wurde.

Die Befüll- und/oder Entnahmevorrichtung kann mit dem Büstenhalter und/oder der Patientin verbunden werden, bevor oder nachdem sie mit der Brustprothese verbunden wird. Beispielsweise kann vorgesehen sein, dass die Befüll- und/oder Entnahmevorrichtung an einer geeigneten Stelle zwischen einem Träger des Büstenhalters und der Haut der Patientin eingeklemmt wird, um diesen beim Einlegen der Prothese und ggf. auch bereits beim Anbinden der noch nicht eingelegten Prothese in geeigneter Position zu halten.

Im Rahmen des vorliegenden Verfahrens kann das Volumen nur einer Brustprothese angepasst werden. Die Brustprothese wird zu diesem Zweck auf einer Seite des Büstenhalters eingelegt und deren Größe wird durch Befüllen oder Entleeren des Fluidraums angepasst, bis deren Größe der Größe der anderen, beispielsweise gesunden Brust entspricht.

Es kann auch vorgesehen sein, dass im Rahmen des vorliegenden Verfahrens die Volumina zweier Brustprothesen angepasst werden, beispielsweise im Falle zweier amputierter Brüste oder einer von der Trägerin gewünschten Größenänderung. In diesem Fall werden Brustprothesen in beide Seiten des Büstenhalters eingelegt und deren Größen werden nacheinander, gegebenenfalls in mehreren Sequenzen durch Befüllen oder Entleeren des Fluidraums angepasst.

Weitere Einzelheiten und Vorteile der Erfindung ergeben sich aus den nachfolgend anhand der Figuren beschriebenen Ausführungsbeispielen. In den Figuren zeigen:
- Figur 1:: eine schematische Darstellung einer im Rahmen des erfindungsgemäßen Verfahrens verwendbaren volumenanpassbaren Brustprothese;
- Figur 2:: eine schematische Darstellung einer Patientin mit einem Büstenhalter und einer Brustprothese auf der rechten Seite, auf der linken Seite und in beiden Seiten des Büstenhalters;
- Figur 3:: eine bildliche Darstellung eines Ablaufs einer Ausführungsvariante eines erfindungsgemäßen Verfahrens; und
- Figur 4:: ein zugehöriges Flussdiagramm;
- Figur 5:: eine bildliche Darstellung eines Ablaufs einer weiteren Ausführungsvariante eines erfindungsgemäßen Verfahrens; und
- Figur 6:: ein zugehöriges Flussdiagramm.

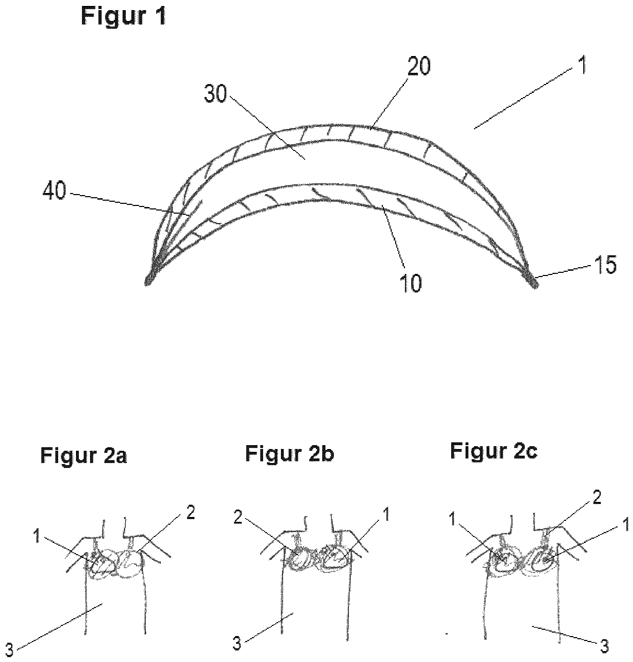

Die in Figur 1 dargestellte Brustprothese 1 mit anpassbarem Volumen kann im Rahmen eines erfindungsgemäßen Verfahrens verwendet werden. Die Brustprothese 1 umfasst einen ersten Schalenkörper 10 an der der Trägerin zugewandten Unterseite der Prothese 1 sowie einen damit umlaufend verbundenen zweiten Schalenkörper 20 an der der Trägerin abgewandten Oberseite der Prothese 1. Bei beiden Schalenkörpern 10 und 20 handelt es sich um Folienbeutel, die mit einer quervernetzten Zwei-Komponenten-Silikonkautschuk-Masse gefüllt sind. Die Folienbeutel sind jeweils aus zwei Kunststofffolienstücken gefertigt, die entlang der gemeinsamen Umfangsfläche miteinander verschweißt sind.

Die Schalenkörper 10 und 20 sind ihrerseits entlang einer umlaufenden Schweißnaht 15 derart verbunden, dass zwischen ihnen ein Fluidraum 30 gebildet wird, der beispielsweise mit Luft, aber auch mit einer Flüssigkeit gefüllt werden kann. Das Volumen der Brustprothese 1 kann durch Befüllung und Entleerung des Fluidraums 30 angepasst werden.

Um eine nachträgliche, d.h. nach der Herstellung erfolgende Befüllung und Entleerung des Fluidraums 30 im Rahmen des erfindungsgemäßen Verfahrens zu ermöglichen, umfasst die Brustprothese 1 einen Ventilschlauch 40 aus einem flexiblen Kunststoffmaterial, der ein flaches Flatterventil umfasst, die Schweißnaht 15 in radiale Richtung durchdringt und dabei zwischen den Schalenkörpern 10 und 20 eingeschweißt ist. Der Ventilschlauch 40 reicht nicht lediglich bis zum Ende der Schweißnaht 15, sondern ragt frei, d.h. ohne mit einem der Schalenkörper 10 oder 20 verbunden zu sein, über die Schweißnaht 15 hinaus in den Fluidraum 30 hinein. Der in den Fluidraum 30 ragende Abschnitt des Ventilschlauchs 40 ist also frei im Fluidraum 30 beweglich.

Um ein Zusammenhaften der Kunststoff-Innenoberflächen der beiden Schalenkörper 10 und 20 aufgrund beispielsweise elektrostatischer Wechselwirkung und mithin ein unerwünschtes Verkleben des Fluidraums 30 zu verhindern, ist der Fluidraum 30 mit einer kleineren Menge an Silikon-Öl befüllt.

Figur 2 zeigt einen Basisablauf eines erfindungsgemäßen Verfahrens für die rechte (Figur 2a), linke (Figur 2b) und beidseitige (Figur 2c) Volumenanpassung mittels eines Fluids von einer oder zwei Brustprothesen 1. Dabei ist vorgesehen, dass die volumenanpassbare Brustprothese 1 zwischen Haut und Cup des Büstenhalters 2 gelegt werden kann. Sofern der Büstenhalter 2 integrierte Taschen aufweist, kann die Prothese 1 auch in diese gelegt werden. Die volumenanpassbare Prothese 1 kann in dieser Position im Rahmen eines erfindungsgemäßen Verfahrens durch die Anwenderin 3 oder eine weitere Person im Volumen durch ein Fluid stufenlos vergrößert oder verkleinert bis das gewünschte Volumen, etwa nach Empfinden der Anwenderin 3, erreicht wird.

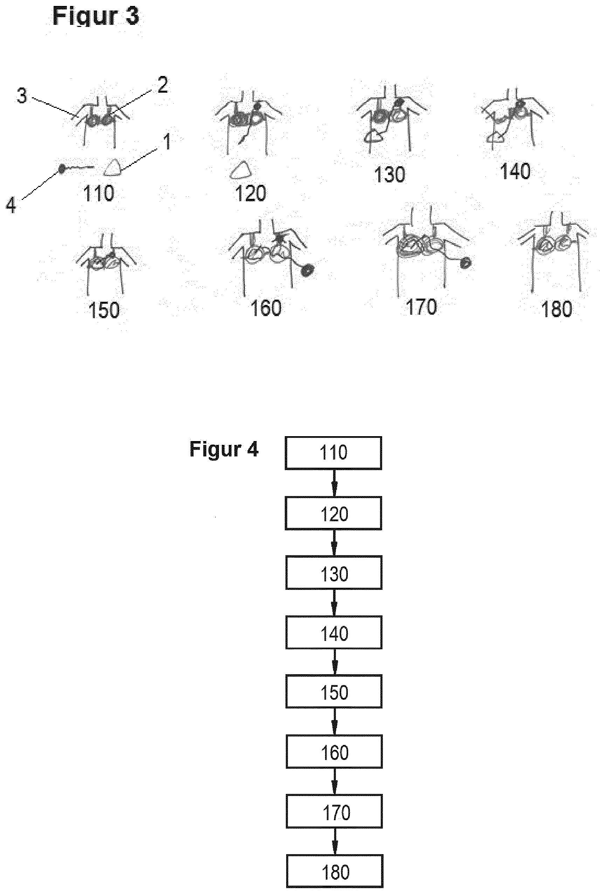

Figuren 3-4 zeigen eine bildliche Darstellung eines Ablaufs einer Ausführungsvariante eines erfindungsgemäßen Verfahrens und ein dazugehöriges Flussdiagramm. In einem ersten Schritt 110 legt sich die Anwenderin 3, welche einen Büstenhalter 2 trägt, die volumenanpassbare Brustprothese 1 und ein geeignetes Pumpzubehör 4 beispielsweise umfassend eine Pumpe, einen Schlauch und eine Nadel zurecht. In einem zweiten Schritt 120 wird das Pumpzubehör 4 an einer geeigneten Position zwischen Büstenhalter 2 und Haut der Patientin 3 befestigt, beispielsweise zwischen den Trägern des Büstenhalters 2 und der Haut der Patientin 3 eingeklemmt. In einem weiteren Schritt 130 wird dann das freie Ende des Pumpzubehörs 4, also beispielsweise die Nadel mit der volumenanpassbaren Brustprothese 1 verbunden, indem beispielsweise die Nadel in den Ventilschlauch gesteckt wird. In einem nächsten Schritt 140 wird an der Seite des Büstenhalters 2, an welcher die Brustprothese 1 eingesetzt werden soll (in diesem Fall aus Sicht der Trägerin rechts), der Büstenhalter 2 geöffnet und der Brustbereich frei gemacht. Die volumenanpassbare Brustprothese 1 wird in Schritt 150 dann zwischen Haut der Patientin 3 und dem Cup des Büstenhalters 2 gelegt. Im Falle eines Büstenhalters 2 mit integrierten Taschen kann die Brustprothese 1 auch in diese gelegt werden. Der Büstenhalter wird dann wieder verschlossen und in die normale Trageposition gebracht. Im Folgenden wird in einem Schritt 160 der eingeklemmte Bereich des Pumpzubehörs 4 mit einer freien Hand der Anwenderin 3 abgenommen. In Schritt 170 vergrößert oder verkleinert die Anwenderin 3 dann mittels des Pumpzubehörs 4 das Volumen der Brustprothese. Das erfindungsgemäße Verfahren endet bei Schritt 180 mit der Finalisierung des Vergrößerungs- bzw. Verkleinerungsvorgangs, wenn die gewünschte Größe erreicht ist.

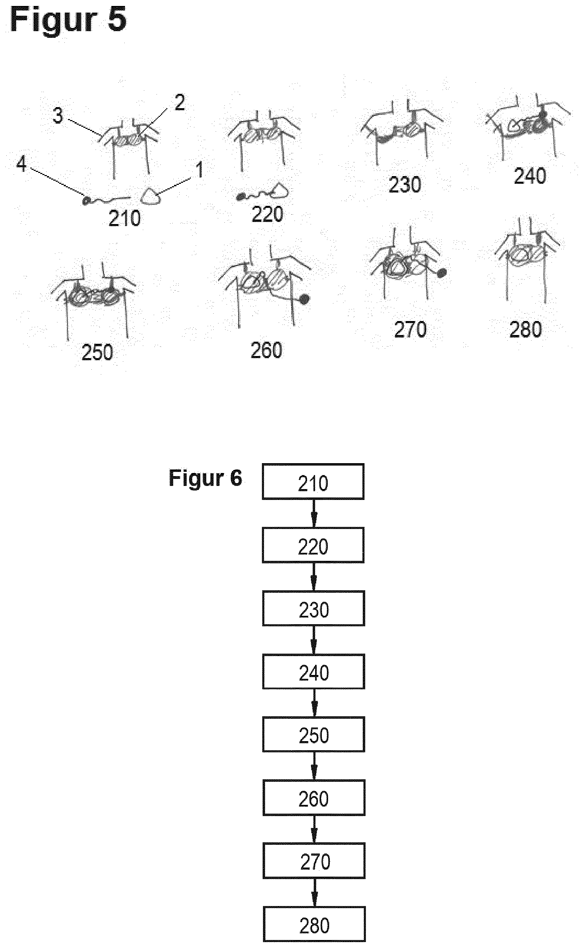

Figuren 5-6 zeigen eine bildliche Darstellung eines Ablaufs einer weiteren Ausführungsvariante eines erfindungsgemäßen Verfahrens und ein dazugehöriges Flussdiagramm. In einem ersten Schritt 210 dieser Ausführungsvariante legt sich die Anwenderin 3, welchen einen geeigneten Büstenhalter 2 trägt, die volumenanpassbare Brustprothese 1 und ein geeignetes Zubehör 4 umfassend eine Pumpe, einen Schlauch und eine Kanüle zurecht. In einem zweiten Schritt 220 verbindet die Anwenderin 3 das Pumpzubehör 4 mit der Brustprothese 1, beispielsweise durch Einstecken der Nadel in den Ventilschlauch. In einem nachfolgenden Schritt 230 wird der Büstenhalter 2 dann an der Seite (rechts aus Sicht der Trägerin 3), an der die Brustprothese 1 eingesetzt werden soll, geöffnet und der Brustbereich frei gemacht. Im Rahmen des Schrittes 240 wird das freie Ende des Pumpzubehörs 4 sodann an einer geeigneten Position zwischen Büstenhalter 2 und Haut der Patientin 3 befestigt, beispielsweise zwischen den Trägern des Büstenhalters 2 und der Haut der Patientin 3 eingeklemmt. Davor, danach oder gleichzeitig wird die Brustprothese 1 auf die freie Brustseite gelegt. In Schritt 250 wird der Büstenhalter 1 dann wieder geschlossen und in die normale Trageposition gebracht. Die folgenden Schritte 260-280 entsprechen den Schritten 160-180 der Ausführungsform gemäß Figuren 3-4.

Die Volumenanpassung mittels des geeigneten Pumpzubehörs 4 kann im Rahmen beider gezeigten Verfahrensführungen beispielsweise auch durch die Anwenderin 3 selbst ohne Hilfsperson vor einem Spiegel erfolgen.

## Patentansprüche

1. Verfahren zur Volumenanpassung einer Brustprothese (1), wobei die Brustprothese (1) einen ersten Schalenkörper (10), einen damit umlaufend verbundenen zweiten Schalenkörper (20) und einen zwischen den Schalenkörpern angeordneten Fluidraum (30) aufweist, wobei der Fluidraum (30) durch einen mit einem Ventil (40) versehenen Durchgang von der Außenseite her zugänglich ist, wobei das Verfahren die folgenden Schritte aufweist:
(a) Einlegen der Brustprothese (1) in einen von einer Patientin getragenen Büstenhalter (2);
(b) Befüllen des Fluidraums (30) mit einem Fluid oder Entnehmen eines Fluids aus dem Fluidraum (30), während die Brustprothese (1) in den von der Patientin getragenen Büstenhalter (2) eingelegt ist.

2. Verfahren nach Anspruch 1, **dadurch gekennzeichnet, dass** es sich bei dem Fluid zur Volumenanpassung um ein Gas, insbesondere um Luft handelt.

3. Verfahren nach Anspruch 1, **dadurch gekennzeichnet, dass** es sich bei dem Fluid zur Volumenanpassung um eine Flüssigkeit, insbesondere um eine nachträglich vernetzbare viskose Flüssigkeit handelt.

4. Verfahren nach Anspruch 3, **dadurch gekennzeichnet, dass** das Verfahren ferner einen Schritt des nachträglichen Vernetzens der in den Fluidraum (30) eingefüllten Flüssigkeit umfasst.

5. Verfahren nach einem der vorhergehenden Ansprüche, **dadurch gekennzeichnet, dass** sich innerhalb des Fluidraums (30) ein von dem in Schritt (b) einzufüllenden Fluid zur Volumenanpassung verschiedenes, zusätzliches Medium befindet, welches die Adhäsionsneigung der gegenüberliegenden Innenoberflächen der Schalenkörper (10, 20) verringert.

6. Verfahren nach einem der vorhergehenden Ansprüche, **dadurch gekennzeichnet, dass** es sich bei dem Durchgang um einen Ventilschlauch (40) handelt, der vorzugsweise in radialer Richtung durch den Verbindungsbereich zwischen den beiden Schalenkörpern (10, 20) verläuft.

7. Verfahren nach Anspruch 6, **dadurch gekennzeichnet, dass** zum Befüllen oder Entnehmen des Fluidraums eine Nadel in den Ventilschlauch gesteckt wird und das Fluid zur Volumenanpassung durch diese Nadel in den Fluidraum (30) gedrückt wird.

8. Verfahren nach Anspruch 7, **dadurch gekennzeichnet, dass** der Ventilschlauch (40) an seiner Innenseite eine Stufe umfasst, an der sich dessen Innendurchmesser in Richtung zum Fluidraum (30) hin verringert, und dass vorzugsweise die Nadel eine korrespondierende Stufe an der Außenseite aufweist, an der sich deren Innendurchmesser in Richtung zum Fluidraum (30) hin verringert.

9. Verfahren nach einem der vorhergehenden Ansprüche, **dadurch gekennzeichnet, dass** die Brustprothese (1) vor Schritt (a) oder zwischen den Schritten (a) und (b) mit einer Befüll- und/oder Entnahmevorrichtung verbunden wird.

10. Verfahren nach Anspruch 9, **dadurch gekennzeichnet, dass** die Befüll- und/oder Entnahmevorrichtung mit dem Büstenhalter (1) und/oder der Patientin verbunden wird, bevor oder nachdem sie mit der Brustprothese (1) verbunden wird.

## Claims

1. A method for the volume adaptation of a breast prosthesis (1), wherein the breast prosthesis (1) has a first shell body (10), a second shell body (20) peripherally connected thereto, and a fluid space (30) arranged between said shell bodies, wherein the fluid space (30) is accessible from the outside through a passage provided with a valve (40), wherein the method comprises the following steps:
(a) inserting the breast prosthesis (1) into a brassiere (2) worn by a patient;
(b) filling the fluid space (30) with a fluid or removing a fluid from the fluid space (30) while the breast prosthesis (1) is inserted into the brassiere (2) worn by the patient.

2. A method in accordance with claim 1, **characterized in that** the fluid for volume adaptation is a gas, in particular air.

3. A method in accordance with claim 1, **characterized in that** the fluid for volume adaptation is a liquid, in particular a subsequently crosslinkable viscous liquid.

4. A method in accordance with claim 3, **characterized in that** the method furthermore includes a step of the subsequent crosslinking of the liquid filled into the fluid space (30).

5. A method in accordance with one of the preceding claims, **characterized in that** an additional medium is present within the fluid space (30) that is different from the fluid for volume adaptation to be filled in step (b) and that reduces the adhesive tendency of the oppositely disposed inner surfaces of the shell bodies (10, 20).

6. A method in accordance with one of the preceding claims, **characterized in that** the passage is a valve tube (40) that preferably extends in the radial direction through the connection region between the two shell bodies (10, 20).

7. A method in accordance with claim 6, **characterized in that** a needle is placed into the valve tube to fill or empty the fluid space; and **in that** the fluid for volume adaptation is pressed through this needle into the fluid space (30).

8. A method in accordance with claim 7, **characterized in that** the valve tube (40) comprises a step at its inner side at which its inner diameter reduces in the direction toward the fluid space (30); and **in that** the needle preferably has a corresponding step at the outer side at which its inner diameter reduces in the direction toward the fluid space (30).

9. A method in accordance with one of the preceding claims, **characterized in that** the breast prosthesis (1) is connected to a filling and/or removal device prior to step (a) or between steps (a) and (b).

10. A method in accordance with claim 9, **characterized in that** the filling and/or removal device is connected to the brassiere (1) and/or to the patient before it after it is connected to the breast prosthesis (1).

## Revendications

1. Procédé d'adaptation du volume d'une prothèse mammaire (1), dans lequel la prothèse mammaire (1) présente un premier corps cupuliforme (10), un deuxième corps cupuliforme (20) relié à celui-ci en périphérie et un espace fluidique (30) disposé entre les corps cupuliformes, dans lequel l'espace fluidique (30) est accessible depuis le côté extérieur par un passage pourvu d'une soupape (40), dans lequel le procédé présente les étapes suivantes :
(a) l'insertion de la prothèse mammaire (1) dans un soutien-gorge (2) porté par une patiente ;
(b) le remplissage de l'espace fluidique (30) avec un fluide ou l'évacuation d'un fluide hors de l'espace fluidique (30), tandis que la prothèse mammaire (1) est insérée dans le soutien-gorge (2) porté par la patiente.

2. Procédé selon la revendication 1, **caractérisé en ce que** le fluide destiné à adapter le volume est un gaz, en particulier de l'air.

3. Procédé selon la revendication 1, **caractérisé en ce que** le fluide destiné à adapter le volume est un liquide, en particulier un liquide visqueux réticulable ultérieurement.

4. Procédé selon la revendication 3, **caractérisé en ce que** le procédé comprend en outre une étape de la réticulation ultérieure du liquide transvasé dans l'espace fluidique (30).

5. Procédé selon l'une quelconque des revendications précédentes, **caractérisé en ce qu'**un milieu supplémentaire différent du fluide à transvaser dans l'étape (b) se trouve à l'intérieur de l'espace fluidique (30), lequel réduit la tendance à l'adhésion des surfaces intérieures opposées des corps cupuliformes (10, 20).

6. Procédé selon l'une quelconque des revendications précédentes, **caractérisé en ce que** le passage est un tuyau flexible de soupape (40), qui s'étend de préférence dans une direction radiale à travers la zone de liaison entre les deux corps cupuliformes (10, 20).

7. Procédé selon la revendication 6, **caractérisé en ce que** pour remplir ou évacuer l'espace fluidique, une aiguille est piquée dans le tuyau flexible de soupape et le fluide est poussé par ladite aiguille dans l'espace fluidique (30) pour l'adaptation du volume.

8. Procédé selon la revendication 7, **caractérisé en ce que** le tuyau flexible de soupape (40) comprend sur son côté intérieur un étage, sur lequel le diamètre intérieur de celui-ci se réduit en direction de l'espace fluidique (30), et que de préférence l'aiguille présente un étage correspondant sur le côté extérieur, sur lequel le diamètre intérieur de celui-ci se réduit en direction de l'espace fluidique (30).

9. Procédé selon l'une quelconque des revendications précédentes, **caractérisé en ce que** la prothèse mammaire (1) est reliée à un dispositif de remplissage et/ou d'évacuation avant l'étape (a) ou entre les étapes (a) et (b).

10. Procédé selon la revendication 9, **caractérisé en ce que** le dispositif de remplissage et/ou d'évacuation est relié au soutien-gorge (1) et/ou à la patiente avant qu'il ne soit relié à la prothèse mammaire ou après qu'il a été relié à la prothèse mammaire (1).
